# EUROPÄISCHE PATENTANMELDUNG

(11) **EP 2 033 624 A2**
(43) Veröffentlichungstag der Anmeldung: **11.03.2009**
(21) Anmeldenummer: 08014890.1
(22) Anmeldetag: 22.08.2008
(51) Int. Cl.: A61K 8/44, A61K 8/46, A61Q 5/02, A61Q 19/10

(54) **Wässrige Konzentrate aus Isethionat, Taurat und Betain**

(30) Priorität: 30.08.2007 DE 102007040909
(71) Anmelder: Clariant International Ltd., 4132 Muttenz (CH)
(72) Erfinder: Loeffler, Matthias, 65510 Idstein (DE)
(74) Vertreter: Paczkowski, Marcus

(57) **Zusammenfassung**

Gegenstand der Erfindung sind wässrige Konzentrate enthaltend, bezogen auf die gesamte Zusammensetzung,
A) 0,1 bis 8,0 Gew.-%, vorzugsweise 3,0 bis 7,0 Gew.-% eines oder mehrerer Isethionate der Formel (1)

RCOOCH₂CH₂SO₃M (1)

worin
R
eine lineare oder verzweigte Alkylgruppe oder Alkenylgruppe und
M
ein Gegenion, ist,

B) 0,1 bis 8,0 Gew.-% eines oder mehrerer Taurate der Formel (2)

R¹CON(CH₃)CH₂CH₂SO₃X (2)

worin R¹ und X die gleiche Bedeutung wie R und M haben
und
C) 0,1 bis 40,0 Gew.-% eines oder mehrerer Alkylbetaine der Formel (3) 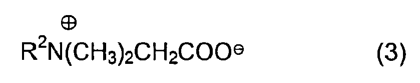 worin R² die gleiche Bedeutung wie R hat, mit der Maßgabe, dass die Konzentration der Summe aller Tenside mit der Formel 1, 2 und 3 mehr als 20 Gew.-% beträgt.

Diese Konzentrate sind bei Raumtemperatur fließfähig und sind transparent sowie farblos.

## Beschreibung

Die Erfindung betrifft hochkonzentrierte, bei Raumtemperatur fließfähige transparente und farblose, wässrige Konzentrate enthaltend Isethionate, Taurate und Alkylbetain.

Isethionate und Taurate sind als milde Tenside mit weiter Verbreitung in kosmetischen Mitteln bekannt.

Sodium Methyl Cocoyl Taurat ist ein anionisches Tensid, welches aufgrund guter Hautverträglichkeit und guten Schaumeigenschaften insbesondere als Cotensid in schäumenden Gesichtsreinigungsmitteln eingesetzt wird. Taurate sind jedoch nur gering in Wasser löslich. Die Löslichkeit von Sodium Methyl Cocoyl Taurat beträgt in Wasser 10 g/l (20 °C). Im Gegensatz zu den sehr viel besser wasserlöslichen Tensiden, wie z. B. Natrium Laureth Sulfate oder Cocamidopropylbetain (die als transparente, dünnflüssige 30 %ige wässrige Lösung z. B. unter den Clariant-Handelsnamen Genapol LRO flüssig oder Genagen CAB erhältlich sind) sind Taurate bei vergleichbarer Konzentration in Wasser weder transparent noch flüssig. So ist die typische Handelsform von Sodium Methyl Cocoyl Taurat eine 30 %ige Paste, welche nur schwer aus den Gebinden zu entnehmen ist und zwingend eine heiße Verarbeitung voraussetzt. Beispiel: Hostapon CT paste (Clariant Produkte (Deutschland) GmbH)

Sodium Cocoyl Isethionat ist ein anionisches Tensid, welches aufgrund seiner guten Schaumeigenschaften und insbesondere wegen seiner sehr geringen Löslichkeit in Wasser seit vielen Jahren als Rohstoff bei der Herstellung von Stückseifen (Combobar oder Syndet) verwendet wird. Die Löslichkeit von Sodium Cocoyl Isethionat beträgt in Wasser nur 0,01 % (20 °C). Daher stehen Isethionate auf dem Markt typischerweise als feste Produkte zur Verfügung. Beispiel: Hostapon SCI 85 Pulver. Der feste Rohstoff muss zur Verarbeitung in kosmetischen Mitteln unter hohem Energie- und Zeitaufwand heiß unter Verwendung von Tensiden oder Lösungsmitteln gelöst werden. Durch die zwingend heiße Verarbeitung ist ferner die Einarbeitung von temperaturempfindlichen Additiven, z. B. Vitamine, Farbstoffe und dergleichen erschwert. Häufig tritt bei der Verarbeitung des festen Isethionates ein lästiges Stauben des Rohstoffs auf, was den Aufwand bei der Verwendung weiter erhöht, beispielsweise ist geeignete Schutzkleidung nötig oder es muss in speziell geschlossenen Anlagen gearbeitet werden.
Tensid-Konzentrate enthaltend schwerlösliche Tenside, z. B. Sodium Cocoyl Isethionat in Gegenwart von amphoteren Tensiden sind bekannt. In diesen Konzentraten werden Ethylenglycol-Mono- und/oder -Distearate, die in Wasser im Wesentlichen unlöslich sind, mit Hilfe von geeigneten Tensiden stabil dispergiert, so dass bei Raumtemperatur eine opake Dispersion vorliegt. In US-5 529 721, US-4 777 038 und US-5 560 873 sind solche Konzentrate beschrieben, die auch unter dem Handelsnamen Mirasheen^{®} (Rhone-Poulenc) oder Euperlan (HENKEL) verfügbar sind.

US-5 415 810 beschreibt Mischungen enthaltend amphotere Tenside in Gegenwart von Sodium Cocoyl Isethionat. Es findet sich jedoch kein Hinweis darauf, dass diese Mischungen klar und stabil sind. Vielmehr werden diese Mischungen direkt nach Herstellung in ein kosmetisches Produkt gegeben, in welchem das Isethionat durch zusätzliche bekannte Additive gelöst und stabilisiert wird und in welchem das Isethionat und die anderen Tenside der Mischung mit Wasser verdünnt werden. Ferner wird in US-5 415 810 nicht die Kombination von Sodium Cocoyl Isethionat mit anderen schwerlöslichen Tensiden im Konzentrat erwähnt. Im Gegenteil hierzu wird ausdrücklich empfohlen, dass schwer lösliche Komponenten wir Fettsäuren nicht in den Konzentraten anwesend sein sollen.

US-4 243 549 beschreibt Konzentrate enthaltend anionische und amphotere Tenside, die jedoch im Wesentlichen über 60 Gew.-% Tensid enthalten und somit eine lamellare oder "G" Phase bilden. Zudem lehrt US-4 243 549 die Verwendung von leicht wasserlöslichen Tensiden, wie z. B. Natrium Laureth Sulfate. Schwerlösliche Tenside, und insbesondere Mischungen schwerlöslicher Tenside wie Sodium Cocoyl Isethionat und Sodium Methyl Cocoyl Taurat werden nicht gelehrt.

Andere Literatur (z. B. US-5 372 751, US-5 518 647, EP-692 240) beschreibt die Verwendung von Sodium Cocoyl Isethionat in Kombination mit amphoteren Tensiden und zusätzlichen anionischem Tensid. Die dort beschriebenen Tensidmischungen sind nur bei geringen Tensidkonzentrationen flüssig, bei hohen
Tensidkonzentrationen sind die Tensidmischungen fest oder pastös, und nicht flüssig und klar. Die Kombination von zwei schwerlöslichen anionischen Tensiden (Sodium Cocoyl Isethionat und Sodium Methyl Cocoyl Taurat) in Coco-Betain wird nicht offenbart.

Überraschend wurde nun gefunden, dass wässrige Mischungen aus Isethionaten, Tauraten und Betainen auch bei hohem Tensidgehalt klare, homogene, fließfähige und pumpbare Konzentrate darstellen, die zudem noch über sehr niedrige Farbzahlen verfügen. Ferner wurde gefunden, dass die Verwendung von Cocobetain als amphoteres Tensid die Solubilisierung einer Mischung von sowohl Tauraten als auch Isethionaten in Wasser in einer erheblich höheren Konzentration ermöglicht, als in Abwesenheit des amphoteres Tensides möglich wäre. Ferner wurde gefunden, dass die Anwesenheit von weiteren schwerlöslichen Tensiden, wie z. B. Fettsäuren (die mit typischerweise bis zu 35 % als Nebenbestandteil in Isethionaten vorliegen) den klaren, homogenen, fließfähigen und pumpbaren Zustand der Konzentrate nicht verändert.
Überraschend wurde ferner gefunden, dass die erfindungsgemäßen Tensidkonzentrate bei gleicher Einsatzkonzentration ein besseres Schaumverhalten aufhalten als die Einzelkomponenten. Das Vorliegen einer synergistischen Mischung in den erfindungsgemäßen Tensidkonzentraten wurde durch Schaummessungen (Ross Miles) und Handwaschtests unter Verwendung eines Expertenpanells nachgewiesen.

### Gegenstand der Erfindung sind transparente, fließfähige, farblose wässrige Konzentrate, enthaltend bezogen auf das gesamte Konzentrat

### A) 0,1 bis 8,0 Gew.-%, vorzugsweise 3,0 bis 7,0 Gew.-% eines oder mehrerer Isethionate der Formel (1)

RCOOCH₂CH₂SO₃M (1)

worin
R eine lineare oder verzweigte Alkylgruppe mit 6 bis 30, vorzugsweise 8 bis 22 C-Atomen oder eine lineare oder verzweigte ein- oder mehrfach ungesättigte Alkenylgruppe mit 6 bis 30, vorzugsweise 8 bis 22 C-Atomen ist und
M ein Gegenion, vorzugsweise ein Alkalimetall-, Erdalkalimetall- oder Ammoniumion, ist,

### B) 0,1 bis 8,0 Gew.-%, vorzugsweise 3,0 bis 7,0 Gew.-% eines oder mehrerer Taurate der Formel (2)

R¹CON(CH₃)CH₂CH₂SO₃X (2)

worin
R¹ eine lineare oder verzweigte Alkylgruppe oder Alkenylgruppe mit 6 bis 30, vorzugsweise 8 bis 22 C-Atomen ist und
X ein Gegenion, vorzugsweise ein Alkalimetall-, Erdalkalimetall- oder Ammoniumion, ist, und

### C) 0,1 bis 40,0 Gew.-%, vorzugsweise 10,0 bis 30,0 Gew.-% eines oder mehrerer Alkylbetaine der Formel (3)

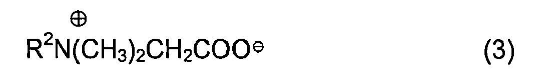

worin
R² eine lineare oder verzweigte Alkylgruppe oder Alkenylgruppe mit 6 bis 30, vorzugsweise 8 bis 22 C-Atomen ist, mit der Maßgabe, dass die Konzentration der Summe aller Tenside mit der Formel 1, 2 und 3 mehr als 20 Gew.-% beträgt.
R, R¹ und R² in den Formeln (1), (2) und (3) sind vorzugsweise die Alkylreste der Cocos- oder Laurylsäure.

Ein besonderer Vorteil der erfindungsgemäßen wässrigen Konzentrate ist, dass sie die per se wasserunlöslichen und festen Tenside Taurat und Isethionat in einer Konzentration klar gelöst enthalten, die ein Mehrfaches über der Löslichkeitsgrenze dieser Komponenten in Wasser liegt. Somit ist es möglich, dass diese Konzentrate ohne einen weiteren Aufheiz- oder Solubilisierungsvorgang in flüssige Endprodukte wie beispielsweise Haarshampoos, Duschbäder oder Flüssigwaschmittel stabil eingearbeitet werden. Es ist ausdrücklich darauf hinzuweisen, dass nicht nur das Konzentrat aus Taurat, Isethionat und Betain sich in wässrigen Tensidmischungen klar und homogen löst, sondern dass das Konzentrat als solches homogen, transparent und niedrigviskos ist. Die Stabilität der erfindungsgemäßen Konzentrate bezieht sich ferner nicht nur auf ein kurzes Zeitfenster nach Herstellung des Konzentrates, sondern umfasst eine dreimonatige Lagerung bei sowohl hohen (40, 45 und 50 °C) als auch niedrigen Temperaturen (- 5, 0 °C).

Eine bevorzugte Ausführungsform der Erfindung sind Zusammensetzungen enthaltend Tenside der Formel (1), (2) und (3), wobei die Gewichtsmenge dieser Tenside zusammen 20 % bis 49 %, bevorzugt 25 % bis 35 %, besonders bevorzugt 28 % bis 32 %, beträgt.

Die erfindungsgemäßen Tensidkonzentrate liegen i.a. vollständig außerhalb der "G" Phase.
Eine ebenso bevorzugte Ausführungsform der Erfindung sind Zusammensetzungen enthaltend Tenside der Formel (1), (2) und (3), wobei der Feststoffgehalt der Zusammensetzungen, bestehend aus den Tensiden der Formel (1), (2) und (3), sowie Fettsäuren und Salzen, insbesondere NaCl, 35 bis 45 %, bevorzugt 40 % beträgt.

Eine weitere bevorzugte Ausführungsform der Erfindung sind Zusammensetzungen enthaltend Tenside der Formel (1), (2) und (3), wobei das Mengenverhältnis von Isethionat gemäß der Formel (1) zu Taurat gemäß der Formel (2) zu Betain gemäß der Formel (3) 0,5 bis 1,5 zu 0,5 bis 1,5 zu 4 beträgt.
Die Transparenz der erfindungsgemäßen Zusammensetzung beträgt bevorzugt max. 10 NTU (Turbidity Standards)
Die Farbzahl der erfindungsgemäßen Zusammensetzung beträgt max. 60 Hazen, bevorzugt max. 30 Hazen, besonders bevorzugt max. 20 Hazen.

Eine besonders bevorzugte Ausführungsform der Erfindung sind wässrige Konzentrate, enthaltend, bezogen auf das gesamte Konzentrat
A) 3,0 bis 7,0 Gew.-% eines oder mehrerer Isethionate der Formel (1)

   RCOOCH₂CH₂SO₃M (1)

   worin
   R der Kohlenwasserstoffrest der Kokossäure ist und
   M ein Alkalimetallion, insbesondere eine Natriumion ist,
B) 3,0 bis 7,0 Gew.-% eines oder mehrerer Taurate der Formel (2)

   R¹CON(CH₃)CH₂CH₂SO₃X (2)

   worin
   R¹ der Kohlenwasserstoffrest der Kokossäure ist und
   X ein Alkalimetallion, insbesondere eine Natriumion ist,
C) 15,0 bis 25,0 Gew.-% eines oder mehrerer Alkylbetaine der Formel (3) 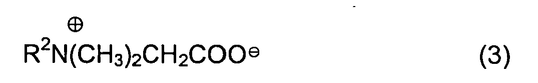 worin
   R² der Kohlenwasserstoffrest der Kokossäure oder der Laurylsäure ist,
D) 5,0 bis 9,0 Gew.-% an Natriumchlorid und
E) 58 bis 62 Gew.-% Wasser und
F) 0,1 bis 2 Gew.-%, bevorzugt 1 Gew.-% Fettsäure, bevorzugt Stearin- oder Kokosfettsäure.

Die erfindungsgemäßen Zusammensetzungen werden zur Herstellung von kosmetischen, dermatologischen oder pharmazeutischen Formulierungen, bevorzugt zur Herstellung von Duschbädern, Shampoos oder Körperreinigungsmitteln, verwendet.

Ein weiterer Gegenstand der Erfindung ist daher die Verwendung der erfindungsgemäßen Zusammensetzungen (Tensidkonzentrate) in kosmetischen, dermatologischen oder pharmazeutischen Formulierungen.

Die kosmetischen, dermatologischen oder pharmazeutischen Formulierungen enthaltend die erfindungsgemäßen Tensidkonzentrate können weitere Tenside, Emulgatoren, kationische Polymere, Verdicker, Filmbildner, antimikrobielle Wirkstoffe, Adstringentien, Antioxidantien, UV-Lichtschutzfilter, Pigmente/Mikropigmente, Gelierungsmittel, sowie weitere in der Kosmetik, Dermatologie oder Pharmazie gebräuchliche Zusätze, wie z. B. Überfettungsmittel, feuchtigkeitsspendende Mittel, Silicone, Stabilisatoren, Konditioniermittel, Glycerin, Konservierungsmittel, Perlglanzmittel, Farbstoffe, Enzyminhibitoren, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistik, Antiallergica, Corticosteroide, Sebostatika), Vitamine, Bisabolol^{®}, Allantoin, Phytantriol^{®}, Panthenol^{®}, AHA-Säuren, Pflanzenextrakte, beispielsweise Aloe Vera und Proteine enthalten.

Als anionische waschaktive Substanzen bzw. Tenside seien vorzugsweise genannt: C₁₀-C₂₀-Alkyl- und Alkylen-carboxylate, Alkylethercarboxylate, Fettalkoholsulfate, , Alkylamidsulfate und -sulfonate, Fettsäurealkylamid-polyglykolethersulfate, Alkansulfate, Alkansulfonate und Hydroxyalkansulfonate, Olefinsulfonate, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuresarkosinate, Sulforicinoleate, Amphoacetate- oder -glycinate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammoniumsowie der analogen Alkylammoniumsalze.

Die Menge der anionischen Tenside in den Formulierungen enthaltend die erfindungsgemäßen Tensidkonzentrate beträgt bevorzugt 1 bis 20 Gew.-%, besonders bevorzugt 5 bis 18 Gew.-%, und insbesondere bevorzugt 10 bis 15 Gew.-%, bezogen auf die fertigen Formulierungen.

Geeignete kationische Tenside sind beispielsweise quartäre Ammonium-Salze wie Di-(C₁₀-C₂₄-Alkyl)-dimethyl-ammonium-chlorid oder -bromid, vorzugsweise Di-(C₁₂-C₁₈-Alkyl)-dimethyl-ammonium-chlorid oder -bromid; C₁₀-C₂₄-Alkyl-dimethyl-ethylammonium-chlorid oder -bromid; C₁₀-C₂₄-Alkyl-trimethyl-ammonium-chlorid oder -bromid, vorzugsweise Cetyl-trimethyl-ammonium-chlorid oder -bromid und C₂₀-C₂₂-Alkyl-trimethyl-ammonium-chlorid oder -bromid; C₁₀-C₂₄-Alkyl-dimethylbenzyl-ammonium-chlorid oder -bromid, vorzugsweise C₁₂-C₁₈-Alkyl-dimethylbenzyl-ammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinolinium-chlorid, -bromid oder -monoalkylsulfat; N-(C₁₂-C₁₈-Alkylpolyoylaminoformylmethyl)-pyridinium-chlorid; N-(C₁₂-C₁₈-Alkyl)-N-methyl-morpholinium-chlorid, -bromid oder -monoalkylsulfat; N-(C₁₂-C₁₈-Alkyl)-N-ethyl-morpholinium-chlorid, -bromid oder -monoalkylsulfat; C₁₆-C₁₈-Alkyl-pentaoxethyl-ammonium-chlorid; Diisobutyl-phenoxyethoxyethyldimethylbenzylammonium-chlorid; Salze des N,N-Diethylamino-ethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Die Menge der kationischen Tenside in den Formulierungen enthaltend die erfindungsgemäßen Tensidkonzentrate beträgt bevorzugt 0,1 bis 10 Gew.-%, besonders bevorzugt 0,2 bis 7 Gew.-%, und insbesondere bevorzugt 0,5 bis 5 Gew.-%, bezogen auf die fertigen Formulierungen.

Als nichtionische Tenside, die als waschaktive Substanzen eingesetzt werden können, kommen vorzugsweise in Betracht Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäureamidpolyethylenglykole; N-Alkyl-, N-Alkoxypolyhydroxyfettsäureamid, insbesondere Fettsäure-N-methylglucamide, Saccharoseester; Polyglykolether, Alkylpolyglycoside, Phosphorsäureester (Mono-, Di- und Triphosphorsäureester ethoxyliert und nicht-ethoxyliert).

Die Menge der nichtionischen Tenside in den Formulierungen enthaltend die erfindungsgemäßen Tensidkonzentrate (z. B. im Falle von Rinse-off-Produkten) liegt bevorzugt im Bereich von 1 bis 20 Gew.-%, besonders bevorzugt 2 bis 10 Gew.-%, und insbesondere bevorzugt 3 bis 7 Gew.-%, bezogen auf die fertigen Formulierungen.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; C₁₂-C₁₈-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxide, z. B. C₁₂-C₁₈-Alkyldimethylaminoxid, Fettsäureamidoalkyl-dimethylaminoxid.

Die Menge der amphoteren Tenside in den Formulierungen enthaltend die erfindungsgemäßen Tensidkonzentrate beträgt bevorzugt 0,5 bis 20 Gew.-% und besonders bevorzugt 1 bis 10 Gew.-%, bezogen auf die fertigen Formulierungen.

Des Weiteren können in den Formulierungen enthaltend die erfindungsgemäßen Tensidkonzentrate schaumverstärkende Co-Tenside aus der Gruppe Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den Formulierungen enthaltend die erfindungsgemäßen Tensidkonzentrate sind Alkylethersulfate, Alkylsulfate, insbesondere Laurylsulfat, Alkylbetaine, Alkylamidopropylbetaine, insbesondere Cocoamidopropylbetain, Amphoacetate, Acylglutamate, insbesondere Natriumcocoylglutamat, Alkylethersulfosuccinate, insbesondere Di-natriumlaureth-sulfosuccinat, und Cocosfettsäurediethanolamid.

Die Gesamtmenge der in den Formulierungen enthaltend die erfindungsgemäßen Tensidkonzentrate eingesetzten Tenside beträgt vorzugsweise 1 bis 20 Gew.-%, besonders bevorzugt 5 bis 18 Gew.-%, und insbesondere bevorzugt 10 bis 15 Gew.-%, bezogen auf die fertigen Formulierungen.

Als kationische Polymere eignen sich vorzugsweise die unter der INCl-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (Salcare^{®} SC95), PVP-dimethylaminoethylmethacrylat-Copolymer, Guarhydroxypropyltriammoniumchloride, sowie Calciumalginat und Ammoniumalginat.

Des Weiteren können vorzugsweise eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxy-propyldiethylentriamin; Polyaminopolyamid und kationische Chitinderivate, wie beispielsweise Chitosan.
Die Menge an kationischen Polymeren in den Formulierungen kann vorzugsweise im Bereich von 0,1 bis 10 Gew.-%, besonders bevorzugt im Bereich von 0,2 bis 5 Gew.-%, und insbesondere bevorzugt im Bereich von 0,5 bis 2,5 Gew.-% liegen, bezogen auf die fertigen Formulierungen.

Bevorzugt geeignet als antimikrobielle Wirkstoffe sind Cetyltrimethylammoniumchlorid, Cetylpyridiniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchloro-hydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die Formulierungen enthalten die antimikrobiellen Mittel bevorzugt in Mengen bis 50 Gew.-%, besonders bevorzugt in Mengen von 0,01 bis 10 Gew.-%, und insbesondere bevorzugt in Mengen von 0,1 bis 10 Gew.-%, bezogen auf die fertigen Formulierungen.

Vorteilhafte Formulierungen enthalten ein oder mehrere Antioxidantien.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den Formulierungen beträgt vorzugsweise 0,001 bis 30 Gew.-%, besonders bevorzugt 0,05 bis 20 Gew.-%, und insbesondere 1 bis 10 Gew.-%, bezogen auf die fertigen Formulierungen.

Als Pigmente/Mikropigmente können vorzugsweise mikrofeines Titandioxid, Glimmer-Titanoxid, Eisenoxide, Glimmer-Eisenoxid, Zinkoxid, Siliziumoxide, Ultramarinblau, Chromoxide, eingesetzt werden.

Weitere Zusatzstoffe können Siliconverbindungen sein, vorzugsweise Dimethylpolysiloxan, Methyl-phenylpolysiloxane, cyclische Silicone sowie amino-, fettsäure-, alkohol-, polyether-, epoxy-, fluor- und/oder alkylmodifizierte Siliconverbindungen, beispielsweise Alkylsilicone SilCare^{®} Silicone 41M10, SilCare^{®} Silicone 41 M15, SilCare^{®} Silicone 41 M20, SilCare^{®} Silicone 41 M30 (Clariant), Alkyltrimethicone SilCare^{®} 31 M30, SilCare^{®} 31 M40, SilCare^{®} 31 M50, SilCare^{®} 31 M60 (Clariant), Phenyltrimethicone SilCare^{®} 15M30, SilCare^{®} 15M40, SilCare^{®} 15M50, SilCare^{®} 15M60 (Clariant), Polyalkylarylsiloxane und Polyethersiloxan-Copolymere.

Die Formulierungen enthaltend die erfindungsgemäßen Tensidkonzentrate können die oben genannten Siliconverbindungen vorzugsweise in den Mengen von 0,1 bis 20 Gew.-%, besonders bevorzugt 0,2 bis 15 Gew.-%, insbesondere bevorzugt 0,5 bis 10 Gew.-%, bezogen auf die fertigen Formulierungen, enthalten.

Als fungizide Wirkstoffe können vorzugsweise Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, ltraconazole, Terconazole, Naftifine und Terbinafine, Zn-Pyrithion und Octopirox^{®} in den Mengen von 0,05 bis 5 Gew.-%, bevorzugt 0,1 bis 3 Gew.-%, und besonders bevorzugt 0,2 bis 2 Gew.-%, bezogen auf die fertigen Formulierungen, eingesetzt werden.

Die Formulierungen enthaltend die erfindungsgemäßen Tensidkonzentrate können vorteilhaft mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen, abgemischt werden.

Als perlglanzgebende Verbindungen bevorzugt sind Fettsäuremonoalkanolamide, Fettsäuredialkanolamide, Monoester oder Diester von Alkylenglykol, insbesondere des Ethylenglykols und/oder Propylenglykols oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art. In den erfindungsgemäßen Formulierungen sind als perlglanzgebende Komponente besonders bevorzugt Ethylenglykoldistearat und Polyethylenglykoldistearat mit 3 Glykoleinheiten.

Als feuchtigkeitsspendende Substanz stehen vorzugsweise Isopropylpalmitat, Glycerin und/oder Sorbitol zu Verfügung, die bevorzugt in den Mengen von 0,1 bis 50 Gew.-% eingesetzt werden, bezogen auf die fertigen erfindungsgemäßen Formulierungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als Konservierungsmittel in Betracht kommen vorzugsweise Phenoxyethanol, Parabene, Pentandiol oder Sorbinsäure. Sie werden vorzugsweise in Mengen von 0,001 bis 5 Gew.-%, besonders bevorzugt von 0,01 bis 3 Gew.-%, und insbesondere bevorzugt von 0,1 bis 2 Gew.-%, bezogen auf die fertigen erfindungsgemäßen Formulierungen, eingesetzt.

Als Farbstoffe können die für kosmetische, dermatologische und pharmazeutische Zwecke geeigneten und zugelassenen Substanzen verwendet werden.

Der Gesamtanteil der Hilfs- und Zusatzstoffe kann 1 bis 50 Gew.-% und vorzugsweise 5 bis 40 Gew.-%, bezogen auf die fertigen Formulierungen, betragen. Die Herstellung der Formulierungen kann durch übliche Kalt- oder Heißprozesse erfolgen.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Die Formulierungen enthaltend die erfindungsgemäßen Tensidkonzentrate sind vorzugsweise auf einen pH-Wert im Bereich 2 bis 12 und besonders bevorzugt auf einen pH-Wert im Bereich 3 bis 8 eingestellt.

In einer weiteren besonders bevorzugten Ausführungsform der Erfindung handelt es sich bei den Formulierungen um Formulierungen mit einem klaren Aussehen.

Die erfindungsgemäßen Tensidkonzentrate und Zusammensetzungen zeichnen sich durch ein sehr gutes Schaumverhalten aus und bewirken ein größeres Schaumvolumen.

Im Ross Miles Test und Handwaschtest wurden die Schaumeigenschaften von einzelnen Tensiden als auch von erfindungsgemäßen Tensidkonzentraten geprüft. Als Gesamttensidkonzentration wurde jeweils 1,0 %, 0,1 % und 0,03 % gewählt, der pH Wert wurde auf pH 7.0 eingestellt und Wasser mit 15°d.H. verwendet. Es wurde die Schaummenge nach 30 und nach 300 Sekunden abgelesen.

Tabellen 1 bis 4:
Schaumhöhe (mm) der erfindungsgemäßen Zusammensetzung im Vergleich zu den Einzelkomponenten

### Hostapon^{®} CT Paste (Sodium Methyl Cocoyl Taurate)

| Gesamttensidkonzentration | Zeit | |
|---|---|---|
| | 30 sec. | 300 sec. |
| 1 % | 240 | 240 |
| 0,1 % | 220 | 220 |
| 0,03 % | 170 | 170 |

### Genagen^{®} KB (Coco-Betaine)

| Gesamttensidkonzentration | Zeit | |
|---|---|---|
| | 30 sec. | 300 sec. |
| 1% | 260 | 240 |
| 0,1 % | 210 | 190 |
| 0,03 % | 180 | 170 |

### Hostapon SCI 85 (Sodium Cocoyl Isethionate)

| Gesamttensidkonzentration | Zeit | |
|---|---|---|
| | 30 sec. | 300 sec. |
| 1% | 250 | 240 |
| 0,1 % | 50 | 5 |
| 0,03 % | 20 | 5 |

Erfindungsgemäßes Tensidkonzentrat bestehend aus
20 % Coco-Betaine
5 % Sodium Methyl Cocoyl Taurate
5 % Sodium Cocoyl Isethionate

| Gesamttensidkonzentration | Zeit | |
|---|---|---|
| | 30 sec. | 300 sec. |
| 1 % | 280 | 275 |
| 0,1 % | 255 | 250 |
| 0,03 % | 215 | 215 |

Das erfindungsgemäße Tensidkonzentrat ergibt eine höhere Schaummenge, die auch nach längerer Wartezeit stabiler ist als bei Verwendung der einzelnen Tenside in gleicher Konzentration.

Die erfindungsgemäßen Tensidkonzentrate und Zusammensetzungen sind farblos bis sehr schwach gefärbt und zeichnen sich durch eine niedrige Farbzahl aus:

Erfindungsgemäße Tensidkonzentrate wurden nach der Pt-Co-/Apha-/Hazen-Farbbewertung bewertet. Die drei Bezeichnungen sind in unterschiedlichen Anwendungsbereichen gebräuchlich, beziehen sich aber auf das gleiche Verfahren. Die Farbbewertung ergänzt die Farbbewertung mit Jod-Vergleichslösungen für schwache Färbungen.

Das Prinzip der Farbbewertung besteht darin, die Proben visuell in genormten Gefäßen mit in der Konzentration abgestuften gelben Standardlösungen zu vergleichen. Für die Apha-/Hazen-/Pt-Co Farbzahl wird nach einem Vorschlag von A. Hazen aus dem Jahre 1892 eine saure Lösung von Kalium-hexachloroplatinat(IV) und Kobalt(II)chlorid eingesetzt. Den Vergleichslösungen wird eine Farbzahl entsprechend ihrem Platingehalt in mg/I (Bereich ist 0-500) zugeordnet. Diese Lösungen sind auch kommerziell erhältlich.

Mehrere internationale und nationale Normen beschreiben das Vorgehen genauer, z. B.:
BS 5339:76 (1993) Measurement of Colour on Hazen Units/Platinum-Cobalt Scale)
DIN 53409 Bestimmung der Hazen-Farbzahl (APHA-Verfahren)
DIN ISO 6271 Einstufung der Farbe nach der Platin Kobalt Skala

A: erfindungsgemäßes Tensidkonzentrat bestehend aus
   20 % Coco-Betaine
   5 % Sodium Methyl Cocoyl Taurate
   5 % Sodium Cocoyl Isethionate
   Hazen Farbzahl: 5
B: erfindungsgemäßes Tensidkonzentrat bestehend aus
   10 % Coco-Betaine
   10 % Sodium Methyl Cocoyl Taurate
   10 % Sodium Cocoyl Isethionate
   Hazen Farbzahl: 18
C: Referenzmischung Miracare^{®} UM-140 bestehend aus
   Sodium Cocoyl Isethionate and Sodium Lauroamphoacetate and Sodium Methyl Cocoyl Taurate and Propylene Glycol and Sodium Xylenesulfonate
   Hazen Farbzahl: 380
D: Referenzmischung Miracare MS-2 bestehend aus:
   PEG-80 Sorbitan Laurate and Sodium Trideceth Sulfate and PEG-150 Distearate and Cocamidopropyl Hydroxysultaine and Disodium Lauroamphodiacetate and Sodium Laureth-13 Carboxylate
   Hazen Farbzahl: 360
E: Referenzmischung Miracare MP-35 bestehend aus:
   Sodium Laureth Sulfate and Cocamide DEA and Cocamidopropyl Betaine and Citric Acid
   Hazen Farbzahl: 290

Die nachfolgenden Beispiele und Anwendungen sollen die Erfindung näher erläutern, ohne sie jedoch darauf zu beschränken (bei allen Prozentangaben handelt es sich um Gew.-%).

Als erfindungsgemäße Zusammensetzung wurde Tensidkonzentrat A verwendet bestehend aus:
20 % Coco-Betaine
5 % Sodium Methyl Cocoyl Taurate
5 % Sodium Cocoyl Isethionate

### Beispiele

### Beispiel 1: Pflegeschampoo

| | | | |
|---|---|---|---|
| | | | |
| A | Genapol^{®} LRO fl. | (Clariant) | 33.30 % |
| | Laurethsulfate, Na-salz | | |
| | Duftstoff | | 0.40 % |
| | | | |
| B | Wasser | | 20.00 % |
| | | | |
| | Ucare Polymer^{®} JR 400 | | 0.30 % |
| | Polyquaternium-10 | | |
| | | | |
| C | Wasser | | ad 100 % |
| | Allantoin | (Clariant) | 0.30 % |
| | Allantoin | | |
| | | | |
| D | Tensidkonzentrat A | | 20.00 % |
| | Coco Betain, Natrium Cocoyl Isethionat, | | |
| | Natrium Methyl Cocoyl Taurat | | |
| | Farbstofflösung | | q.s. |
| | Konservierungsmittel | | q.s. |
| | D-Panthenol | | 0.50 % |
| | | | |
| E | Zitronensäure (25 %) | | q.s. |

### Herstellweise

I Mischen der Komponenten A
II Lösen von B bei 80 °C
III Lösen von C bei 80 °C
IV Nacheinander Einrühren von II und III in I
V Zugabe von D zu IV
VI Einstellen des pH mit E auf 5.5

### Beispiel 2: klares Antischuppenschampoo

| | | | |
|---|---|---|---|
| A | Genapol^{®} LRO fl. | (Clariant) | 38.90 % |
| | Laurethsulfat, Na-Salz | | |
| | Duftstoff | | 0.40 % |
| | Octopirox^{®} | (Clariant) | 0.50 % |
| | Pirocton Olamin | | |
| | | | |
| B | Wasser | | ad 100 % |
| | Tensidkonzentrat A | | 16.00 % |
| | Coco Betain, Natrium Cocoyl Isethionat, | | |
| | Natrium Methyl Cocoyl Taurat | | |
| | Farbstofflösung | | q.s. |
| | | | |
| C | Zitronensäure (25 %) | | q.s. |

Herstellweise:
I Mischen der Komponenten A
II Nacheinander Zugabe der Komponenten B zu I
III Einstellen des pH mit C auf 5.5

### Beispiel 3: Pflegeschampoo

| | | |
|---|---|---|
| A | Ucare Polymer^{®} JR 400 | 0.40 % |
| | Polyquaternium-10 | |
| | Wasser | ad 100 % |
| | | |
| B | Tensidkonzentrat A | 8,00 % |
| | Coco Betain, Natrium Cocoyl Isethionat, Natrium Methyl Cocoyl Taurate | |
| | Genapol^{®} LRO fl. | 35.00 % |
| | Laurethsulfat, Na-Salz | |
| | Belsil DMC 6032 | 0.50 % |
| | Dimethicone PEG-6 Acetate | |
| | Genagen^{®} KB | 3.50 % |
| | Coco Betain | |
| | | |
| C | Glycerin | 2.00 % |
| | Sorbitol | 2.00 % |
| | Mackpro SLP | 0,50 % |
| | Quaternium-79 Hydrolyzed Soy Protein | |
| | | |
| D | Zitronensäure 25 %ig | q.s. |
| | | |
| E | Konservierungsmittel | q.s. |
| | Farbstofflösung | q.s. |
| | Duftstoff | 0,30% |
| | | |
| F | Natriumchlorid | 1,50% |

### Herstellweise:

I Lösen von A bei 80 °C
II Mischen der Komponenten von B
III Nacheinander Zugabe von C zu I
IV Einrühren von III in II; Rühren bis zum Aufklaren
V Einstellen des pH mit D auf pH 5.5
VI Zugabe von E
VII Einstellen der Viskosität mit F

### Beispiel 4: Gesichtsreinigungsmittel

| | | |
|---|---|---|
| A | Genapol^{®} LRO fl. (Clariant) | 11.10 % |
| | Laurethsulfat, Na-Salz | |
| | Duftstoff | 0.40 % |
| B | Wasser | ad 100 % |
| | Tensidkonzentrat A | 23.30 % |
| | Coco Betain, Natrium Cocoyl Isethionat, | |
| | Natrium Methyl Cocoyl Taurat | |
| | Farbstofflösung | q.s. |
| | Konservierungsmittel | q.s. |
| | | |
| C | Zitronensäure | q.s. |

### Herstellweise:

I Mischen der Komponenten von A
II Zugabe von B zu I
III Einstellen des pH mit C

### Beispiel 5: Anti-Akne Gesichtsreinigungsmittel

| | | | |
|---|---|---|---|
| A | Genapol^{®} LRO fl. | (Clariant) | 16.00 % |
| | Laurethsulfat, Na-Salz | | |
| | Duftstoff | | 0.40 % |
| | Octopirox^{®} | (Clariant) | 0.50 % |
| | Pirocton Olamin | | |
| | | | |
| B | Allantoin | (Clariant) | 0.30 % |
| | Allantoin | | |
| | | | |
| C | Wasser | | ad 100 % |
| | | | |
| D | Tensidkonzentrat A | | 16.00 % |
| | Coco Betain, Natrium Cocoyl Isethionat, | | |
| | Natrium Methyl Cocoyl Taurat | | |
| | Farbstofflösung | | q.s. |
| E | Zitronensäure (25 %) | | q.s. |

### Herstellweise

I Mischen der Komponenten von A
II Lösen von B in erwärmten C; Abkühlen unter Rühren
III Zugabe von II zu I
IV Zugabe von D zu III
V Einstellen auf pH 5,5 mit E.

## Patentansprüche

1. Wässrige Konzentrate enthaltend, bezogen auf die gesamte Zusammensetzung,
A) 0,1 bis 8,0 Gew.-%, vorzugsweise 3,0 bis 7,0 Gew.-% eines oder mehrerer Isethionate der Formel (1)
RCOOCH₂CH₂SO₃M (1)
worin
R eine lineare oder verzweigte Alkylgruppe mit 6 bis 30, vorzugsweise 8 bis 22 C-Atomen oder eine lineare oder verzweigte ein- oder mehrfach ungesättigte Alkenylgruppe mit 6 bis 30, vorzugsweise 8 bis 22 C-Atomen ist und
M ein Gegenion, vorzugsweise ein Alkalimetall-, Erdalkalimetall- oder Ammoniumion, ist,
B) 0,1 bis 8,0 Gew.-%, vorzugsweise 3,0 bis 7,0 Gew.-% eines oder mehrerer Taurate der Formel (2)
R¹CON(CH₃)CH₂CH₂SO₃X (2)
worin
R¹ eine lineare oder verzweigte Alkylgruppe mit 6 bis 30, vorzugsweise 8 bis 22 C-Atomen oder eine lineare oder verzweigte ein- oder mehrfach ungesättigte Alkenylgruppe mit 6 bis 30, vorzugsweise 8 bis 22 C-Atomen ist und
X ein Gegenion, vorzugsweise ein Alkalimetall-, Erdalkalimetall- oder Ammoniumion, ist, und
C) 0,1 bis 40,0 Gew.-%, vorzugsweise 10,0 bis 30,0 Gew.-% eines oder mehrerer Alkylbetaine der Formel (3) 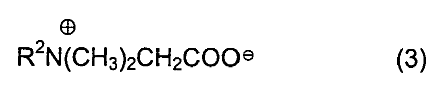 worin
R² eine lineare oder verzweigte Alkylgruppe mit 6 bis 30, vorzugsweise 8 bis 22 C-Atomen oder eine lineare oder verzweigte ein- oder mehrfach ungesättigte Alkenylgruppe mit 6 bis 30, vorzugsweise 8 bis 22 C-Atomen ist, mit der Maßgabe, dass die Konzentration der Summe aller Tenside mit der Formel 1, 2 und 3 mehr als 20 Gew.-% beträgt.

2. Wässrige Konzentrate nach Anspruch 1, enthaltend solche Verbindungen der Formeln (1), (2) und (3), worin R, R¹ oder R² den Alkylrest der Kokos- oder Laurylsäure ist.

3. Wässrige Konzentrate nach Anspruch 1, wobei die Gewichtsmenge aller Tenside der Formeln (1), (2) und (3) 20 bis 49 % beträgt.

4. Wässrige Konzentrate nach Anspruch 1, wobei die Gewichtsmenge aller Tenside der Formeln (1), (2) und (3) 25 bis 35 % beträgt.

5. Wässrige Konzentrate nach Anspruch 1, wobei die Gewichtsmenge aller Tenside der Formeln (1), (2) und (3) 28 bis 32 % beträgt.

6. Wässrige Konzentrate nach Anspruch 1, wobei der Feststoffgehalt, bestehend aus den Tensiden der Formeln (1), (2) und (3) sowie Fettsäuren und Salzen 35 bis 45 Gew.-% beträgt.

7. Wässrige Konzentrate nach Anspruch 1, wobei das Mengenverhältnis der Verbindungen der Formeln (1), (2) und (3) 0,5 bis 1,5 zu 0,5 bis 1,5 zu 4 beträgt.

8. Wässrige Konzentrate nach Anspruch 1, **gekennzeichnet durch** eine Farbzahl von maximal 60 Hazen, bevorzugt maximal 30 Hazen, insbesondere maximal 20 Hazen.

9. Wässrige Konzentrate nach einem oder mehreren der Ansprüche 1 bis 8, **gekennzeichnet durch** eine Transparenz von maximal 10 NTU

10. Wässrige Konzentrate enthaltend, bezogen auf die gesamte Zusammensetzung,
A) 3,0 bis 7,0 Gew.-% eines oder mehrerer Isethionate der Formel (1)
RCOOCH₂CH₂SO₃M (1)
worin
R der Kohlenwasserstoffrest der Kokossäure ist und
M ein Alkalimetallion ist,
B) 3,0 bis 7,0 Gew.-% eines oder mehrerer Taurate der Formel (2)
R¹CON(CH₃)CH₂CH₂SO₃X (2)
worin
R¹ der Kohlenwasserstoffrest der Kokossäure ist und
X ein Alkalimetallion ist,
C) 15,0 bis 25,0 Gew.-% eines oder mehrerer Alkylbetaine der Formel (3) 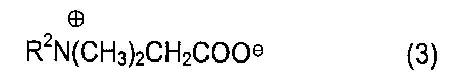 worin
R² der Kohlenwasserstoffrest der Kokossäure ist,
D) 5,0 bis 9,0 Gew.-% an Natriumchlorid und
E) 58 bis 62 Gew.-% Wasser und
F) 0,1 bis 2 Gew.-%, bevorzugt 0,5 - 1 Gew.-% Fettsäure, bevorzugt Stearin-oder Kokosfettsäure.

11. Kosmetische, dermatologische oder pharmazeutische Formulierungen, enthaltend ein wässriges Konzentrat nach einem oder mehreren der Ansprüche 1 bis 10.
